# EUROPEAN PATENT APPLICATION

(11) **EP 0 788 799 A2**
(43) Date of publication of application: **13.08.1997**
(21) Application number: 97100852.9
(22) Date of filing: 21.01.1997
(51) Int. Cl.: A61K 38/09

(54) **LHRH-Antagonists in the treatment of fertility disorders**

(30) Priority: 07.02.1996 US 11282
(71) Applicant: ASTA Medica Aktiengesellschaft, D-01277 Dresden (DE)
(72) Inventor: Engel, Jürgen Prof. Dr., 63755 Alzenau (DE); Bouchard, Philippe Bouchard, Prof. Dr., F-75014 Paris (FR); Frydman, René, Prof. Dr., F-75006 Paris (FR); Diedrich, Klaus Prof. Dr. med., D-23627 Gross-Sarau (DE); Devroey, Paul, Prof. Dr., B-9100 Aalst (BE)

(57) **Abstract**

This inventions relates to the preparation of a medicament to be applied in the field of treating infertility disorders with or without assisted reproduction techniques. In particular the improvement is directed to use an LH-RH Antagonist preferabely Cetrorelix for preparation of an medicament applied in the method of treating infertility disorders by inducing follicle growth by administration of exogenous gonadotropins and in administering the LH-RH Antagonist which contains an amount of LH-RH Antagonist as low as only to suppress endogenous LH but the FSH secretion is maintained at a natural level and the individual estrogen development is not affected. When using the preparation, the follicle development must not be in each case externally stimulated (e.g. by the addition of gonadotropins) but can be maintened by endogenous gonadotropins. Advantageously the preparation can be given in the range of 0.1 to 5 mg of Cetrorelix / day during a multiple dosing posoilogy.

## Description

### Background:

The reasons for unsuccessful attempts to establish a pregnancy can equally be attributed to male and female fertility disorders. Today many different assisted reproduction techniques are available which often are used for the induction of multiple and synchronuous follicular growth in order to obtain fertilizable oocytes.

The current standard-treatment to induce multiple follicular development is based on the administration of high doses of HMG (Human Menopausal Gonadotropin), resulting in ovarian hyperstimulation. After having reached a suitable degree of maturation, then ovulation is induced by the administration of HCG (Human Chorion-Gonadotropin) in order to obtain a sufficient number of oocytes and at a time when the clinic-infrastructure is prepared for the subsequent actions, which are: recovery of oocytes by abdominal or transvaginal puncture, intracorporal or extracorporal fertilization of oocytes by different techniques and embryo replacement into the uterus. Routinely, the beginning pregnancy is supported by additional administrations of HCG or progesterone.

Today this treatment is applied to clinical conditions of male and female infertility. Complications that are frequently observed during the hyperstimulation procedure are:
A: premature surges of luteinizing hormone (LH) at a premature maturation state with a rupture of the follicles induced a subsequent cancellation of the treatment occuring in about 25% of the patients and
B: ovarian hyperstimulation syndroms induced by exogenous gonadotropines which in severe cases require hospitalisation and are life-threatening.

In order to avoid premature LH-surges, today LHRH-Agonists are used as a co-medication. By continued administration of these drugs a complete suppression of endogenous gonadotropins by desensitation of pituitary cells and down-regulation of there receptors. Subsequently, the gonadotropin levels can be controlled by exogenous injection and the pituitary is refractory to the stimulation of LH-release by increasing levels of estradiol. Disadvantages are represented by a long treatment period until the suppression and down-regulation occurs and by the occurrens of estrogene withdrawal symptomes and disturbance of the normal menstrual cycle and also by frequent hormone determinations in order to evaluate the time of onset of suppression. Subsequently high dose HMG treatment is initiated for ovarian stimulation.

The pathogenesis of the above mentioned hyperstimulation syndrom is not completely understood, but today it is thought to be associated with the use of HCG for ovulation induction and luteal phase support.

One recent approach within the treatment of infertility is represented by the use of the LHRH antagonist Cetrorelix (INN). In first clinical trials, short term treatment with Cetrorelix resulted in a complete avoidance of premature LH surges during stimulated cycles and a significantly reduced need of HMG. Due to the immediate suppression of gonadotropins by this antagonist the unwanted stimulatory phase and also the withdrawal of estrogene produced by the agonists can be avoided and the duration of treatment can be shortened significantly. In addition, it was recently shown that a single injection of an antagonist, given in the mid follicular phase can adequately suppress premature LH surges.

### Invention

Despite of the improvements described above, the treatment modalities all aim at treating the patients with the highest possible dose of exogenous gonadotropins in order to hyperstimulate multiple follicular development and hence, the treatment regimens still need to be improved in terms of reducing severe adverse events, improving patient's compliance and reducing costs. Recent data obtained with Cetrorelix showed that there are surprising new advantages for the treatment of male and female infertility.
In animal experiments and clinical studies with Cetrorelix, it was possible to induce an arrest of the normal, unstimulated follicular growth by multiple or single injections. The effect was observed with doses being extremly low. This resulted in the new possibility to manipulate the time of ovulation during a normal, not exogen gonadotropin- stimulated cycle, without affecting the viability of the growing follicle. In case of inadequate folliculare growth related to treatment with LHRH-Antagonists, low dose and short term administration of gonadotrophin or other trophic compounds will compensate these effects. Subsequently, by stopping the LHRH-Antagonist treatment, it is possible to let the normal ovulation occur or to induce it by exogeneous manipulation if indicated. Then ovulation induction can be performed by administration of standard HCG or by administration of LHRH and by LHRH agonistic analogs.

The described treatment alternatives are completely new, since they only exist if the preceeding treatment for LH-surge-control was performed with an LHRH-Antagonist. In animal and clinical studies with Cetrorelix it was shown that the responsiveness of the pituitary to LHRH or agonistic analogs is preserved under these conditions. Contrary to this situation, the pituitary can not respond after agonistic pretreatment for LH-surge control due to receptor down-regulation. In addition, the possible use of other ovulation inducing agents than HCG, results in a reduced incidence of ovarian hyperstimulation syndroms.

On the basis of the described results, for the first time it is possible to use normal, non-gonadotropin-stimulated cycles for assisted reproduction techniques, including sperm injections, by determining the time of ovulation by the duration and dose of Cetrorelix given. Especially in conjuction with the method of ICSI (Intra-Cytoplasmatic-Sperm-Injection) this antagonist-dependent treatment modality facilitates the inclusion of in-(sub-)fertile males into this kind of fertility treatment. Due to the direct injection of male gamets capable for fertilization, this method has a high success-rate and hence, allows the harvest of only one follicle for fertilization. In addition, the use of LHRH-Antagonists like Cetrorelix in the descibed manner reliefes the patient from severe ovarian hyperstimulation and significantly reduces the costs of a treatment cycle.

### Example

238 patients were treated with Cetrorelix by subcutaneous injection of Cetrorelix Acetat-Lyophilisat.
134 patients were treated with multiple doses and 104 patients with single or dual doses. The multiple doses are 0.25 mg/ day or higher. The single dose was 3 mg or higher.
No premature LH surge was seen in any patient undergoing controlled ovarian superovulation for assisted reproduction technology (COS/ART) treated with these dosages. Multiple doses were applied for 3 to a maximum of 10 days dependent on follicular development.

As a result 71 pregnancies were obtained = 30.0 %
38 of 134 following the multiple dose regimen = 28.4 %
33 of 104 following the single/dual dosage regimen = 31.7 %

Following treatment 44 babies were born that means 15 following multiple doses and 29 following single/dual doses. 16 pregnancies are still ongoing.
Table shows I this in particular.
The table III shows an absolute prevention of any premature LH surge. Furthermore, FSH secretion is maintained at a natural level and therefore the individual estrogen development is not affected.

The main advantages in controlled ovarian stimulation (COS/ART) with Cetrorelix are :
1. New therapeutic principle
   ⇒ For COS/ART
   ⇒ In the prevention of premature LH surges Uniform and continuous follicular synchronization Uniform and continuous estradiol development Very low LH-values for optimal follicular development FSH development not affected
2. Short treatment duration of 3 to 7, max. 14 days
   ⇒ Short term exposure during follicular development
   ⇒ Low medication exposure during follicular development
3. No flare-up but immediate hormonal response
4. No pretreatment for 14 to 21 days before start of HMG
5. Fit into normal menstrual cycle
   ⇒ No modification of physiological menstrual cycle pattern
   ⇒ No hormonal withdrawal syndromes before stimulation
6. No or only ultrashort-term residual effects after ovulation induction
7. No residual effects during and following embryo transfer
8. No ovarian cyst formation before start of stimulation
9. Reduction of dose of gonadotropins

Table II (flow chart) shows an example on a typical treatment start and duration of HMG and Cetrorelix in patients to undergo controlled ovarian superovulation for ART.

## Claims

1. Use of an LH-RH Antagonist for preparation of an medicament applied in the method of treating infertility disorders by administering an LH-RH Antagonist and inducing follicle growth by administration of exogenous gonadotropin which contains an amount of LH-RH Antagonist as low as only to suppress endogenous LH but the FSH secretion is maintained at a natural level and the individual estrogen development is not affected.

2. The preparation according to claim 1 for use in a method of treating infertility disorders by administering a LH-RH Antagonist and inducing follicle growth by administration of exogenous gonadotropin wherein the antagonist is Cetrorelix.

3. The preparation according to claim 2 for use in a method wherein the follicle growth is stimulated with other substances than exogenous gonadotropins.

4. The preparation according to claim 2 for use in a method wherein after the inhibition of the action of natural LH caused by the LH-RH Antagonist, preferabely Cetrorelix, the follicle development is not externally stimulated (e.g. by the addition of gonadotropins) but maintened by endogenous gonadotropins.

5. The preparation according to claim 2 wherein the amount of subcutaneously given Cetrorelix is in the range of 0,1 to 5 mg of Cetrorelix / day during a multiple dosing posology.

6. Use of an LH-RH Antagonist for preparation of a medicament for controlled ovarian stimulation in which Cetrorelix is applied starting cycle day 1 to 10, preferabely on day 4 to 8 and ovulation can be induced between *day 8 and 18* of the menstruation cycle.

7. The medicament according to claim 1 wherein the LH-RH Antagonist is given as a single or dual subcutaneous dose in the range of 1 mg to 10 mg, preferabely 2 mg to 6 mg.

8. The medicament for controlled ovarian stimulation in which the LH-RH Antagonist, preferablely Cetrorelix, is applied according to claim 7 starting on cycle day 6 to 10 and ovulation can beinduced between day 9 to 16 of the menstruation cycle.

9. The medicament according to claim 6 whereas the ovulation is induced by rec. LH.

10. The medicament according to claim 6 whereas the ovulation is induced by native LHRH

11. The medicament according to claim 6 whereas the ovulation is induced by a LHRH agonist.

12. The medicament according to claim 6 whereas the ovulation is induced by HCG

13. The medicament according to claim 8 wherein native LHRH or a LHRH agonist are given to avoid luteal phase supplementation in preventing the negative effects of HCG during the luteal phase.

14. The medicament according to claim 8 wherein rec. LH, native LHRH or LHRH agonist are given to avoid hyperstimulation syndrome.
